# EUROPEAN PATENT SPECIFICATION

(11) **EP 1 551 395 B1**
(45) Date of publication and mention of the grant of the patent: **11.07.2007**
(21) Application number: 03758297.0
(22) Date of filing: 06.10.2003
(51) Int. Cl.: A61K 31/4045, A61K 31/422, A61K 31/404, A61K 31/405, A61K 31/445, A61K 31/216, A61K 31/48, A61K 31/192, A61K 31/18, A61K 31/635, A61K 31/506, A61P 25/06

(54) **5-HT 1B/1D RECEPTOR AGONISTS FOR THE TREATMENT OF HEADACHE RESULTING FROM ADMINISTERING AN ENDOTHELIN RECEPTOR ANTAGONIST**
5-HT 1B/1D REZEPTOR AGONISTEN ZUR BEHANDLUNG VON KOPFSCHMERZ DER AUS DER VERABREICHUNG VON ENDOTHELIN REZEPTOR ANTAGONISTEN RESULTIERT
AGONISTES RECEPTEURS DE 5-HT 1B/1D POUR LE TRAITEMENT DE MAUX DE TETE PRODUITS PAR L'ADMINISTRATION D'UN ANTAGONISTE RECEPTEUR D'ENDOTHELINE

(30) Priority: 09.10.2002 GB 0223367
(43) Date of publication of application: 13.07.2005
(73) Proprietor: AstraZeneca AB, 151 85 Södertälje (SE)
(72) Inventor: CURWEN, Jon Owen, Macclesfield, Cheshire SK10 4TG (GB); HUGHES, Andrew Mark, Macclesfield, Cheshire SK10 4TG (GB); JOHNSTONE, Donna, Macclesfield, Cheshire SK10 4TG (GB); MORRIS, Clive Dylan, Macclesfield, Cheshire SK10 4TG (GB)
(74) Representative: Bill, Kevin
(86) International application number: PCT/GB2003/004338
(87) International publication number: WO 2004/032922

(56) References cited:
- WO-A-02/067987

## Description

The present invention relates to the use of a 5-HT_{1B/1D} receptor agonist, or a pharmaceutically acceptable salt thereof in the treatment or prevention of headache that results from administering an endothelin receptor antagonist, or a pharmaceutically acceptable salt thereof. The present invention further relates to a combination comprising an endothelin receptor antagonist, or a pharmaceutically acceptable salt thereof, and a 5-HT_{1B/1D} receptor agonist, or a pharmaceutically acceptable salt thereof.

Cancer affects an estimated 10 million people worldwide. This figure includes incidence, prevalence and mortality. More than 4.4 million cancer cases are reported from Asia, including 2.5 million cases from Eastern Asia, which has the highest rate of incidence in the world. By comparison, Europe has 2.8 million cases, North America 1.4 million cases, and Africa 627,000 cases.

In the UK and US, for example, more than one in three people will develop cancer at some point in their life. Cancer mortality in the U.S. is estimated to account for about 600,000 a year, about one in every four deaths, second only to heart disease in percent of all deaths, and second to accidents as a cause of death of children 1-14 years of age. The estimated cancer incidence in the U.S. is now about 1,380,000 new cases annually, exclusive of about 900,000 cases of non-melanotic (basal and squamous cell) skin cancer.

Cancer is also a major cause of morbidity in the UK with nearly 260,000 new cases (excluding non-melanoma skin cancer) registered in 1997. Cancer is a disease that affects mainly older people, with 65% of cases occurring in those over 65. Since the average life expectancy in the UK has almost doubled since the mid nineteenth century, the population at risk of cancer has grown. Death rates from other causes of death, such as heart disease, have fallen in recent years while deaths from cancer have remained relatively stable. The result is that 1 in 3 people will be diagnosed with cancer during their lifetime and 1 in 4 people will die from cancer. In people under the age of 75, deaths from cancer outnumber deaths from diseases of the circulatory system, including ischaemic heart disease and stroke. In 2000, there were 151,200 deaths from cancer. Over one fifth (22 per cent) of these were from lung cancer, and a quarter (26 per cent) from cancers of the large bowel, breast and prostate.

Worldwide, the incidence and mortality rates of certain types of cancer (of stomach, breast, prostate, skin, and so on) have wide geographical differences which are attributed to racial, cultural, and especially environmental influences. There are over 200 different types of cancer but the four major types, lung, breast, prostate and colorectal, account for over half of all cases diagnosed in the UK and US. Prostate cancer is the fourth most common malignancy among men worldwide, with an estimated 400,000 new cases diagnosed annually, accounting for 3.9 percent of all new cancer cases.

Current options for treating cancers include surgical resection, external beam radiation therapy and / or systemic chemotherapy. These are partially successful in some forms of cancer, but are not successful in others. Recently, endothelin A receptor antagonists have been identified as potentially of value in the treatment of cancer (Cancer Research, 56, 663-668, February 15th, 1996 and Nature Medicine, Volume 1, Number 9, September 1999, 944-949).

The endothelins are a family of endogenous 21 amino acid peptides comprising three isoforms, endothelin-1, endothelin-2 and endothelin-3. The endothelins are formed by cleavage of the Trp²¹-Va²² bond of their corresponding proendothelins by an endothelin converting enzyme. The endothelins are among the most potent vasoconstrictors known and have a characteristic long duration of action. They exhibit a wide range of other activities including cell proliferation and mitogenesis, extravasation and chemotaxis, and also interact with a number of other vasoactive agents.

The endothelins are released from a range of tissue and cell sources including vascular endothelium, vascular smooth muscle, kidney, liver, uterus, airways, intestine and leukocytes. Release can be stimulated by hypoxia, shear stress, physical injury and a wide range of hormones and cytokines. Elevated endothelin levels have been found in a number of disease states in man including cancers.

However, the administration of endothelin receptor antagonists is known to result in headaches in man. Administration to man of Bosentan (Tracleer^{™}) at the recommended dose of 125-250mg bid, this endothelin receptor antagonist induces headaches in 22% of subjects as reported in the prescribing information (Physicians Desk Reference, 2002). The incidence of headache in cancer patients receiving therapy with another endothelin receptor antagonist, Atrasentan, is cited at 50-100% of subjects, at doses of 20mg and above (Carducci MA, Nelson JB, Bowling MK, Rogers T, Eisenberger MA, Sinibaldi V, Donehower R, Leahy TL, Carr RA, Isaacson JD, Janus TJ, Andre A, Hosmane BS, Padley RJ; "Atrasentan, an endothelin-receptor antagonist for refractory adenocarcinomas: safety and pharmacokinetics" J Clin Oncol. 2002 Apr 15; 20(8): 2171-80). With respect to this latter citation, "the headache began with the initiation of therapy and resolved after several days of atrasentan treatment... These headaches were controlled, as necessary with standard analgesic therapy".

ZD4054 is an endothelin receptor antagonist which has recently commenced clinical testing, and which also induces headaches in man. Based on citations by Carducci (Carducci, 2002) a number of "standard analgesic therapies" were tried, including paracetamol, ibuprofen and codeine to treat the ZD4054 induced headaches. Despite standard analgesic therapy, several subjects still reported persistent headache. Surprisingly, administration of the 5HT_{1B/1D} agonist zolmitriptan, licensed for the treatment of migraine, proved effective in reducing the intensity of ZD4054 induced headaches in some subjects in which standard analgesic therapy had already failed.

During migraine excessive cerebrovascular dilation and neurogenic inflammatory processes are considered to contribute to pain. The 5-HT_{1B/1D}-receptors mediate cerebrovascular vasoconstriction and inhibit neurogenic inflammation. 5-HT_{1B/1D} receptor agonists are beneficial in the treatment (including prophylaxis) of disease conditions wherein vasodilation and neurogenic inflammation in the cerbrovascular bed is indicated.

Combinations of a 5-HT₁ receptor agonist with an analgesic, and an anti- emetic and/or a gastroprokinetic agent are described in WO02/067987.

The present inventors have surprisingly found that an endothelin antagonist induced headache is responsive to migraine therapy. As stated above standard therapy for the headache is a traditional analgesic which works by intercepting the pain mechanism itself. The 5-HT_{1B/1D} receptor agonists work by vasoconstriction of the cerebral arteries and the fact that the endothelin antagonist induced headache is sensitive to this vasoconstriction is surprising. This unexpected finding could not have been anticipated and is a novel approach to the treatment of the endothelin antagonist induced headache. Furthermore, endothelin antagonists have no demonstrable 5HT receptor binding activity.

The present invention relates to the use of a 5-HT_{1B/1D} receptor agonist, or a pharmaceutically acceptable salt thereof in the treatment or prevention of headaches that result from administering an endothelin receptor antagonist, or a pharmaceutically acceptable salt thereof.

According to a further feature of the present invention, there is provided a combination, comprising an endothelin receptor antagonist, or a pharmaceutically acceptable salt thereof, and a 5-HT_{1B/1D} receptor agonist, or a pharmaceutically acceptable salt thereof.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the benefit of the combination.

In one aspect, where a compound or a pharmaceutically acceptable salt thereof, is referred to this refers to the compound only. In another aspect this refers to a pharmaceutically acceptable salt of the compound.

Where the term "headache" is referred to it is to be understood that this term includes headaches, migraines, cluster headaches and headache associated with vascular disorders.

Herein where the treatment of headache occurs it is to be understood that this refers to both the prophylactic use of a 5-HT_{1B/1D} receptor agonist, and the use of a 5-HT_{1B/1D} receptor agonist upon onset of headache.

Where cancer is referred to, particularly it refers to oesophageal cancer, myeloma, hepatocellular, pancreatic, cervical cancer, ewings tumour, neuroblastoma, kaposis sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, renal cancer, brain cancer, lymphoma and leukaemia. More particularly it refers to prostate cancer. In addition, more particularly it refers to SCLC, NSCLC, colorectal cancer, ovarian cancer and / or breast cancer. In addition, more particularly it refers to SCLC. In addition, more particularly it refers to NSCLC. In addition, more particularly it refers to colorectal cancer. In addition, more particularly it refers to ovarian cancer. In addition, more particularly it refers to breast cancer. Furthermore, more particularly it refers to bladder cancer, oesophageal cancer, gastric cancer, melanoma, cervical cancer and / or renal cancer. In addition it refers to endometrial, liver, stomach, thyroid, rectal and / or brain cancer. In another aspect of the invention, the cancer is not melanoma. In another embodiment of the invention, particularly the cancer is in a metastatic state, and more particularly the cancer produces metastases to the bone. In a further embodiment of the invention, particularly the cancer is in a metastatic state, and more particularly the cancer produces skin metastases. In a further embodiment of the invention, particularly the cancer is in a metastatic state, and more particularly the cancer produces lymphatic metastases. In a further embodiment of the invention, the cancer is in a non-metastatic state.

Where the "treatment or prevention of headache" is referred it is to be understood that this refers to the treatment or prevention of headaches and related conditions for example providing pain relief, decreasing nausea, decreasing photophobia and phonophobia.

Suitable compounds, or a pharmaceutically acceptable salt thereof, possessing endothelin receptor antagonist activity include those described in US 5292740, US 5334598, US 5378715, US 5389620, US 5420123, US 5464853, US 5482960, US 5514691, US 5514696, US 5541186, US5543521, US 5559105, US 5571821, US 5780473, US 5962490, US 5965732, US 6080774, US 6420567, US 2002091272(A1), WO 93/08799, WO 93/21219, WO 93/23404, WO 93/25580, WO 94/02474, WO 94/03493, WO 94/14434, WO 94/21259, WO 94/21590, WO 94/24084, WO 94/25013, WO 94/27979, WO 95/03044, WO 95/03295, WO 95/04530, WO 95/04534, WO 95105372, WO 95/05374, WO 95/05376, WO 95/08989, WO 95/12611, WO 95/13262, WO 95/15944, WO 95/15963, WO 96/20177, WO 95/26360, WO 95/26716, WO 95/26360, WO95/26957, WO 95/33748, WO 95/33752, WO 95/35107, WO 96/04905, WO 96/06095, WO 96/07653, WO 96/08483, WO 96/08486, WO 96/08487, WO 96/09818, WO 96/11914, WO 96/11927, WO 96/12706, WO 96/15109, WO 96/19455, WO 96/19459, WO 96/22978, WO 96/23773, WO 96/30358, WO 96/31492, WO 96/33170, WO 96/33190, WO96/40681, WO 97/30045, WO 98/09953, WO 95/08550, WO 98/49162, WO 99/06397, WO 01/49685, WO 02/64573, EP 436189, EP 496452, EP 510526, EP 526708, EP 552417, EP 555537, EP 601386, EP 617001, EP 628569, EP 633259, EP 658548, EP 682016, EP 713875, EP 702012, EP 733626, EP 743307, EP 749964, GB2266890, GB 2275926, GB 2276383, GB 2277446, GB 2295616, DE 4341663, JP 6256261, JP 6122625, JP 7330622, JP 7133254, JP 8059635, JP 7316188, and JP 7258098.

Additional suitable compounds, or a pharmaceutically acceptable salt thereof, possessing endothelin receptor antagonist activity include those described in the J Med Chem papers 1996, 39, 2123-2128; 1997, 40, 3, 322-330; 2001, 44, 1211-1216; 2001**,** 44, 3978-3984.

Further suitable compounds, or a pharmaceutically acceptable salt thereof, possessing endothelin receptor antagonist activity include A-127722, atrasentan (ABT-627), BQ-123, BQ-788, BMS 182874, feloprentan, BSF 420627, FR139317, IPI-950, L-749,329, L-754,142, LU 110896, LU 110897, PD 156707, PD 155080, Ro 46-2005, bosentan (Ro 47-0203), SB 217242, SB 209670, TAK-044, YM598, sitaxsentan (TBC11251), ZD1611, ambrisentan, tezosentan, darusentan, *N*-[[2'-[[(4,5-dimethyl-3-isoxazolyl)amino]sulphonyl]-4-(2-dxazolyl)[1,1'-biphenyl]-2-yl]methyl]-N,3,3-trimethylbutanamide and *N*-(3-methoxy-5-methylpyrazin-2-yl)-2-(4-[1,3,4-oxadiazol-2-yl]phenyl)pyridine-3-sulphonamide (ZD4054).

Further suitable compounds, or a pharmaceutically acceptable salt thereof, possessing endothelin receptor antagonist activity include A-104029, A-158112, A-182086, A-192621, A-201661, A-203719, A-206377, A-207508, A-308165, ABT-306552, ABT-546, BE-18257B, BQ-017, BQ-145, BQ-238, BQ-518, BQ-928, J-104121, J-104132, J-112287, BSF-461314, BMS-187308, BMS-193884, edonentan, IRL-1543, IRL-1722, IRL-1841, TBC-10662, TBC-11040, TBC-11299, TBC-3711, Ro-48-5694, Ro-61790C, VML-588, FR-901367, FR-901533, Ro-46-8443, IPI-616, LU-127043, K-8794, RES 1214-1, RES-1149-1, RES-701-1, RES-701-2. BQ-153, BQ-485, BQ-610, L-744453, L-746072, L-747844, EMD-122801, EMD-122946, EMD-94246, CGP-49941, CGS-27830, IRL-1038, IRL-1666, IRL-3461, PD-102566, PD-152884, PD-155719, PD-156123, PD-156252, PD-159020, PD-159433, PD-160672, PD-160874, PD-162073, PD-163070, PD-166673, PD-155218, CI-1034, PD-142893, PD-145065, PD-151242, PD-159110, PD-161721, PD-163610, PD-164800, RPR-105227, RPR-111613, RPR-110477, Ro-06-2687, Ro-43-1736, Ro-44-9099, Ro-48-5695, SPP-301, 50-235 (Shionogi & Co Ltd), 97-139 (Shionogi & Co Ltd), S-0139, S-1255, SB-255757, SB-215355, SB-234551, SB-247083, SQ-34520, SQ-35469, TAN-2162, T-0201, ATZ-1993, YM-62899 and ZD-2574.

A particular compound possessing endothelin receptor antagonist activity is atrasentan (ABT-627) or a pharmaceutically acceptable salt thereof. A particular compound possessing endothelin receptor antagonist activity is YM598 or a pharmaceutically acceptable salt thereof. A particular compound possessing endothelin receptor antagonist activity is ZD4054 or a pharmaceutically acceptable salt thereof. A particular compound possessing endothelin receptor antagonist activity is ZD1611 or a pharmaceutically acceptable salt thereof.

In another aspect of the invention the endothelin receptor antagonist, or a pharmaceutically acceptable salt thereof, is an endothelin A receptor antagonist. In a further aspect of the invention, the endothelin receptor antagonist, or a pharmaceutically acceptable salt thereof, is an endothelin B receptor antagonist. In an additional aspect of the invention, the endothelin receptor antagonist, or a pharmaceutically acceptable salt thereof, is a mixed endothelin A and B receptor antagonist.

Suitable compounds, or a pharmaceutically acceptable salt thereof, possessing 5-HT_{1B/1D} receptor agonist activity include those described in EP 486666, WO 97/06162 and EP 765322.

Particular classes of 5-HT_{1B/1D} receptor agonists are the triptans, or a pharmaceutically acceptable salt thereof.

Further particular compounds, or pharmaceutically acceptable salts thereof, possessing 5-HT_{1B/1D} receptor agonist activity include zolmitriptan, sumatriptan, eletriptan, frovatriptan, naratriptan, rizatriptan and almotriptan. Particularly the compound, or a pharmaceutically acceptable salt thereof, possessing 5-HT_{1B/1D} receptor agonist activity is zolmitriptan ((S)-4-{{3-[2-(dimethylaminoethyl]-1H-indol-5-yl]methyl]-2-oxazolidinone), or a pharmaceutically acceptable salt thereof.

Further particular compounds, or pharmaceutically acceptable salts thereof, possessing 5-HT_{1B/1D} receptor agonist activity include RU-24969, BMS-181885, GR-46611, MDL-74721, IS-159, L-694247, L-741604, L-772405, L-775606, CGS-12066B, CP-108509, CP-119333, CP-124439, CP-135807, CP-161242, CP-94253, CP-122288, donitriptan, MT-400, parthenolide, GMC-2021, SKF-99101H, SB-205209, SB-236057 and 5-(nonyloxy)tryptamine.

Particular combinations of the present invention include:
- ZD4054, or a pharmaceutically acceptable salt thereof, and zolmitriptan, or a pharmaceutically acceptable salt thereof;
- ZD1611, or a pharmaceutically acceptable salt thereof, and zolmitriptan, or a pharmaceutically acceptable salt thereof;
- atrasentan, or a pharmaceutically acceptable salt thereof, and zolmitriptan, or a pharmaceutically acceptable salt thereof; and
- YM598, or a pharmaceutically acceptable salt thereof, and zolmitriptan, or a pharmaceutically acceptable salt thereof.
- Bosentan, or a pharmaceutically acceptable salt thereof, and zolmitriptan, or a pharmaceutically acceptable salt thereof.
- ZD4054, or a pharmaceutically acceptable salt thereof, and sumatriptan, or a pharmaceutically acceptable salt thereof;
- ZD1611, or a pharmaceutically acceptable salt thereof, and sumatriptan, or a pharmaceutically acceptable salt thereof;
- atrasentan, or a pharmaceutically acceptable salt thereof, and sumatriptan, or a pharmaceutically acceptable salt thereof; and
- YM598, or a pharmaceutically acceptable salt thereof, and sumatriptan, or a pharmaceutically acceptable salt thereof.
- Bosentan, or a pharmaceutically acceptable salt thereof, and sumatriptan, or a pharmaceutically acceptable salt thereof.
- ZD4054, or a pharmaceutically acceptable salt thereof, and naratriptan, or a pharmaceutically acceptable salt thereof;
- ZD1611, or a pharmaceutically acceptable salt thereof, and naratriptan, or a pharmaceutically acceptable salt thereof;
- atrasentan, or a pharmaceutically acceptable salt thereof, and naratriptan, or a pharmaceutically acceptable salt thereof; and
- YM598, or a pharmaceutically acceptable salt thereof, and naratriptan, or a pharmaceutically acceptable salt thereof.
- Bosentan, or a pharmaceutically acceptable salt thereof, and naratriptan, or a pharmaceutically acceptable salt thereof.

Further particular combinations include the four identified immediately above but wherein zolmitriptan is replaced with one of sumatriptan, eletriptan, frovatriptan, naratriptan, rizatriptan or almotriptan.

In any of the combinations described herein, including the use of these combinations, kits or formulations containing them etc., an additional compound or compounds (see below) can optionally be present. The combination of an endothelin receptor antagonist, or a pharmaceutically acceptable salt thereof, and a 5-HT_{1B/1D} receptor agonist, or a pharmaceutically acceptable salt thereof, can optionally be administered in further combination with:
i) an LHRH analogue; and / or
ii) a bisphosphonate.

Herein where the term "LHRH analogue" is used it is to be understood that this refers to any chemical compound, or a pharmaceutically acceptable salt thereof, including small molecules and peptides, which acts as an agonist or antagonist at the LHRH receptor, whether by an interaction with the LHRH binding site or by an allosteric mechanism, i.e. acts at a position on the LHRH receptor different to the LHRH binding site. In one aspect of the invention an "LHRH analogue" refers to an LHRH antagonist or a pharmaceutically acceptable salt thereof. In one aspect of the invention an "LHRH analogue" refers to an LHRH agonist or a pharmaceutically acceptable salt thereof. In a further aspect of the invention an "LHRH analogue" refers to a combination of an LHRH antagonist or a pharmaceutically acceptable salt thereof and an LHRH agonist or a pharmaceutically acceptable salt thereof.

Particular compounds, or pharmaceutically acceptable salts thereof possessing LHRH analogue activity include Azaline B, A-198401, A-75998, A-76154, A-84861, abarelix, AN-152, AN-207, Antide, avorelin, cetrorelix, D-21775, D-23487, D-26344, D-63153, D-85108, degarelix, deslorelin, detirelix, FE 200486, ganirelix, gonadimmune, goserelin, histrelin, leuprolide, leuprorelin, metarelin, nafarelin, NBI-42902 (Neurocrine), Org-30850, PH-45 (Pherin Corp), PTL-03001, ramorelix, RWJ-47428-021, SPD-424, surfagon, T-66 (Matrix Therapeutics Ltd), TAK-013, TAK-810, teverelix, triptorelin acetate, triptorelin pamoate, vomeropherin or ZK-157348.

Particular LHRH analogues are peptides or peptide derivatives.

Examples of particular LHRH agonists include, but are not limited to;
i) buserelin (US Patent 4 024 248)
   (pyr)Glu-His-Trp-Ser-Tyr-D-Ser(Bu')⁶-Leu-Arg-Pro-NHCH₂CH₃
ii) triptorelin (US Patent 4 010 125)
   (pyr)Glu-His-Trp-Ser-Tyr-Trp-Leu-Arg-Pro-Gly-NH₂
iii) leuprorelin (Us Patent 4 005 063)
   (pyr)Glu-His-Trp-Ser-Tyr-D-Leu-Leu-Arg-Pro-NHCH₂CH₃
iv) goserelin (US Patent 4 100 274)
   (pyr)Glu-His-Trp-Ser-Tyr- D-Ser(Bu^{t})⁶-Leu-Arg-Pro-(Azygly)NH₂
v) deslorelin (US Patent 4 659 695)
   (pyr)Glu-His-Trp-Ser-Tyr-D-Trp-Leu-Arg-Pro-NH-CHZ-CHZ-NH2
vi) histerelin (US Patent 4 244 946)
   (pyr)Glu-His-Trp- Ser-Tyr-D-His(Bzl)-Leu-Arg-Pro-NH-CH₂-CH₃
vii) avorelin (US 5 668 254)
   (pyr)Glu-His-Trp-Ser-Tyr-D-Trp(2-Me)-Leu-Arg-Pro-NH-CH₂-CH₂
viii) nafarelin (US Patent 4 234 571)
   (pyr)Glu-His-Trp-Ser-Tyr-D-Nal(2)-Leu-Arg-Pro-NH-CH₂-CH₃;
lutrelin, cystorelin, gonadorelin or detirelix.

Particularly the LHRH agonist is selected from leuprorelin, buserelin, triptorelin and goserelin. More particularly the LHRH agonist is goserelin.

Examples of suitable LHRH antagonists include, but are not limited to, antide, abarelix, antarelix, cetrorelix, azaline, ganirelix and those disclosed in US Patents 5 470 947 (Folkers); 5 413 990 and 5 300 492 (Haviv); 5 371 070 (Koerber); 5 296 468 (Hoeger); 5 171 635 (Janaky); 5 003 011 and 4 431 635 (Coy); 4 992 421 (De); 4 801 577 (Nestor); and 4 851 385, 4 689 396 and 5 843 901 (F.oeske).

Further examples of suitable LHRH antagonists include, but are not limited to the compounds described in WO 02/066477, WO 02066478, WO 02/066459, WO 02/092565, PCT/GB03/003603 and PCT/GB03/003606.

A "bisphosphonate" is a compound, or a pharmaceutically acceptable salt thereof, capable of regulating metal cations content (especially calcium content) in humans and is a compound containing two carbc n phosphorous bonds. For further explanation of the term "bisphosphonate" the readers at:ention is drawn to Endocrine Reviews, 1998, 19(1): 80-100.

Particular bisphosphonates for use in the present invention are selected from tiludronic acid, ibandronic acid, incadronic acid, risedronic acid, zoledronic acid, clodronic acid, neridronic acid, pamidronic acid, alendronic acid, minodronic acid, olpadronic acid, TRK 530, CGP 47072, calcium clodionate or EB 1053. Further particular bisphosphonates for use in the present invention are selected from etidronic acid, PNU-91638, NE-21650, NE-58025, NE-10790 or NE-10446.

Furthermore, in any of the combinations described herein, including the use of these combinations, kits or formulations containing them etc., further or alternative additional compound or compounds (see below) can optionally be present. The combination of an endothelin receptor antagonist, or a pharmaceutically acceptable salt thereof, and a 5-HT_{1B/1D} receptor agonist, or a pharmaceutically acceptable salt thereof, can optionally be administered in further combination with standard analgesics for example:
i) paracetamol;
ii) acetaminophen;
iii) non-steroidal anti inflammatory agents; and
iv) opiates.

Suitable pharmaceutically-acceptable salts include, for example, salts with alkali metal (such as sodium, potassium or lithium), alkaline earth metals (such as calcium or magnesium), ammonium salts, and salts with organic bases affording physiologically acceptable cations, such as salts with methylamine, dimethylamine, trimethylamine, piperidine and morpholine. In addition, for those compounds which are sufficiently basic, suitable pharmaceutically-acceptable salts include, pharmaceutically-acceptable acid-addition salts with hydrogen halides, sulphuric acid, phosphoric acid and with organic acids such as citric acid, maleic acid, methanesulphonic acid and p-toluenesulphonic acid. Alternatively, the compounds may exist in zwitterionic form.

In subjects with ZD4054 induced headache, a dose of 2.5-5mg zolmitriptan administered orally was found to be effective in reducing the severity of headache where standard analgesic therapy had failed.

According to the present invention, there is provided the use of a 5-HT_{1B/1D} receptor agonist, or a pharmaceutically acceptable salt thereof, in the treatment or prevention of headache that results from administering an endothelin receptor antagonist, or a pharmaceutically acceptable salt thereof.

In this aspect of the invention, there is provided the use of a 5-HT_{1B/1D} receptor agonist, or a pharmaceutically acceptable salt thereof, in the manufacture of a medicament for the treatment or prevention of headache that results from administering an endothelin receptor antagonist, or a pharmaceutically acceptable salt thereof, in a warm-blooded animal, such as man.

In a further aspect of the present invention, there is provided a combination, comprising an endothelin receptor antagonist, or a pharmaceutically acceptable salt thereof, and a 5-HT_{1B/1D} receptor agonist, or a pharmaceutically acceptable salt thereof for use as a medicament.

According to a further aspect of the invention there is provided a pharmaceutical composition which comprises an endothelin receptor antagonist, or a pharmaceutically acceptable salt thereof, and a 5-HT_{1B/1D} receptor agonist, or a pharmaceutically acceptable salt thereof, in association with a pharmaceutically acceptable diluent or carrier.

According to a further aspect of the invention there is provided a pharmaceutical composition which comprises an endothelin receptor antagonist, or a pharmaceutically acceptable salt thereof, in association with a pharmaceutically acceptable diluent or carrier, in combination with a pharmaceutical composition which comprises a 5-HT_{1B/1D} receptor agonist, or a pharmaceutically acceptable salt thereof, in association with a pharmaceutically acceptable diluent or carrier.

Therefore according to the present invention, there is provided a method of treating cancer, in a warm-blooded animal, such as man, in need of such treatment which comprises administering to said animal an effective amount of an endothelin receptor antagonist, or a pharmaceutically acceptable salt thereof in combination with an effective amount of a 5-HT_{1B/1D} receptor agonist, or a pharmaceutically acceptable salt thereof.

For the avoidance of doubt, where the treatment of cancer is indicated, it is to be understood that this also refers to the prevention of metastases and the treatment of metastases, i.e. cancer spread. Therefore the combination of the present invention could be used to treat a patient who has no metastases to stop them occurring, or to lengthen the time period before they occur, and to a patient who already has metastases to treat the metastases themselves. Furthermore the treatment of cancer also refers to treatment of an established primary tumour or tumours and developing primary tumour or tumours. In one aspect of the invention the treatment of cancer relates to the prevention of metastases. In another aspect of the invention the treatment of cancer relates to the treatment of metastases. In another aspect of the invention the treatment of cancer relates to treatment of an established primary tumour or tumours or developing primary tumour or tumours. Herein, the treatment of cancer also refers to the prevention of cancer *per se.*

In addition the treatment of cancer also refers to the production of an anti-angiogenic effect in a warm blooded animal.

According to a further aspect of the present invention there is provided a kit comprising an endothelin receptor antagonist, or a pharmaceutically acceptable salt thereof, and a 5-HT_{1B/1D} receptor agonist, or a pharmaceutically acceptable salt thereof; optionally with instructions for use.

According to a further aspect of the present invention there is provided a kit comprising:
a) an endotheiin receptor antagonist, or a pharmaceutically acceptable salt thereof, in a first unit dosage form;
b) a 5-HT_{1B/1D} receptor agonist, or a pharmaceutically acceptable salt thereof; in a second unit dosage form; and
c) container means for containing said first and second dosage forms; and optionally
d) with instructions for use.

According to a further aspect of the present invention there is provided a kit comprising:
a) an endothelin receptor antagonist, or a pharmaceutically acceptable salt thereof, together with a pharmaceutically acceptable diluent or carrier, in a first unit dosage form;
b) a 5-HT_{1B/1D} receptor agonist, or a pharmaceutically acceptable salt thereof, in a second unit dosage form; and
c) container means for containing said first and second dosage forms; and optionally
d) with instructions for use.

According to a further aspect of the invention there is provided a pharmaceutical composition which comprises an endothelin receptor antagonist, or a pharmaceutically acceptable salt thereof, and a 5-HT_{1B/1D} receptor agonist, or a pharmaceutically acceptable salt thereof, in association with a pharmaceutically acceptable diluent or carrier for use in the treatment of cancer.

According to a further aspect of the invention there is provided a pharmaceutical composition which comprises an endothelin receptor antagonist, or a pharmaceutically acceptable salt thereof, in association with a pharmaceutically acceptable diluent or carrier, in combination with a pharmaceutical composition which comprises a 5-HT_{1B/1D} receptor agonist, or a pharmaceutically acceptable salt thereof, in association with a pharmaceutically acceptable diluent or carrier for use in the treatment of cancer.

The pharmaceutical compositions may be in a form suitable for oral administration, for example as a tablet or capsule (conventional or "fast-melt"), for parenteral injection (including intravenous, subcutaneous, intramuscular, intravascular or infusion) as a sterile solution, suspension or emulsion, for topical administration as an ointment or cream, for rectal administration or for intranasal administration including a nasal spray formulation. In general the above compositions may be prepared in a conventional manner using conventional excipients and according to methods generally known in the art of formulation technology. For example formulations of the 5-HT_{1B/1D} receptor agonist zolmitriptan may be prepared according to EP 486666, WO 01/39772, US 5,178,878, US 6,024,981.

For example ZD4054 can be formulated as a tablet using the following excipients:
ZD4054;
Lactose monohydrate (filler);
Croscarmellose sodium (disintegrant);
Povidone (binder);
Magnesium stearate (lubricant);
Hypromellose (film coat component);
Polyethylene glycol 300 (film coat component); and
Titanium dioxide (film coat component).

According to a further aspect of the present invention there is provided a kit comprising an endothelin receptor antagonist, or a pharmaceutically acceptable salt thereof, and a 5-HT_{1B/1D} receptor agonist, or a pharmaceutically acceptable salt thereof; optionally with instructions for use; for use in the treatment of cancer.

According to a further aspect of the present invention there is provided a kit comprising:
a) an endothelin receptor antagonist, or a pharmaceutically acceptable salt thereof, in a first unit dosage form;
b) a 5-HT_{1B/1D} receptor agonist, or a pharmaceutically acceptable salt thereof; in a second unit dosage form; and
c) container means for containing said first and second dosage forms; and optionally
d) with instructions for use;
for use in the treatment of cancer.

According to a further aspect of the present invention there is provided a kit comprising:
a) an endothelin receptor antagonist, or a pharmaceutically acceptable salt thereof, together with a pharmaceutically acceptable diluent or carrier, in a first unit dosage form;
b) a 5-HT_{1B/1D} receptor agonist, or a pharmaceutically acceptable salt thereof, in a second unit dosage form; and
c) container means for containing said first and second dosage forms; and optionally
d) with instructions for use; for use in the treatment of cancer.

According to another feature of the invention there is provided the use of an endothelin receptor antagonist, or a pharmaceutically acceptable salt thereof, in combination with a 5-HT_{1B/1D} receptor agonist, or a pharmaceutically acceptable salt thereof, in the manufacture of a medicament for use in the treatment of cancer, in a warm-blooded animal, such as man.

According to another feature of the invention there is provided the use of an endothelin receptor antagonist, or a pharmaceutically acceptable salt thereof, in combination with a 5-HT_{1B/1D} receptor agonist, or a pharmaceutically acceptable salt thereof, in the treatment of cancer, in a warm-blooded animal, such as man.

According to a further aspect of the present invention there is provided a combination comprising an endothelin receptor antagonist, or a pharmaceutically acceptable salt thereof, and a 5-HT_{1B/1D} receptor agonist, or a pharmaceutically acceptable salt thereof, for use in the treatment of cancer.

Endothelin antagonists are also known to be useful in the treatment of cardiovascular diseases or medical conditions such as hypertension, pulmonary hypertension, congestive heart failure, dyslipidaemia, atherosclerosis, restenosis, acute and chronic renal failure, ischaemic stroke, subarachnoid haemorrhage, intermittent claudication, critical limb ischaemia, asthma, and organ failure after general surgery or translantation. They may also be useful for the treatment of pre-eclampsia, premature labour, myocardial infarction, angina pectoris, dysrrhythmia, cardiogenic and endotoxin shock, diabetes mellitus, Raynaud's disease, scleroderma, Buerger's disease, systemic sclerosis, bronchitis, acute respiratory distress syndrome, liver cirrhosis, osteoporosis, Crohn's disease, ulcerative colitis, irritable bowel syndrome, urinary incontinence, migraine, glaucoma and arthritis. Therefore, the combinations of the present invention will also be useful in the treatment of these diseases.

Furthermore, endothelin antagonists might also be useful in the treatment and/or prophylaxis of pain of different origins and causes, including acute as well as chronic pain states. Examples are pain caused by chemical, mechanical, radiation (including sunburn), thermal (including bums), infectious or inflammatory tissue trauma or cancer, postoperative pain, post-partum pain, the pain associated with joint conditions (such as rheumatoid arthritis and osteoarthritis), pain associated with dental conditions (such as dental caries and gingivitis), myofascial and low back pain, pain associated with bone disorders (such as osteoporosis, hypercalcaemia of malignancy and Paget's disease) and the pain associated with sports injuries and sprains.

Also neuropathic pain conditions of central or peripheral origin could be treated with endothelin antagonists. Examples of these pain conditions are pain associated with trigeminal neuralgia, pain associated with postherpetic neuralgia (PHN), pain associated with diabetic mono/poly neuropathy, pain associated with nerve trauma, pain associated with spinal cord injury, pain associated with central post stroke, pain associated with multiple sclerosis and pain associated with Parkinson's disease.

Other pain states of visceral origin such as caused by ulcer, dysmenorrhea, endometriosis, irritable bowel syndrome, dyspepsia etc. could also be treated or prevented with endothelin antagonists.

Endothelin antagonists might also be useful in the oral treatment of neuropathic or central pain states.

Therefore, as a further aspect of the invention, the combinations of the present invention will also be useful in the treatment of pain, including the pain states listed herein above.

According to a further aspect of the present invention there is provided a combination treatment comprising the administration of an effective amount of an endothelin receptor antagonist, or a pharmaceutically acceptable salt thereof, optionally together with a pharmaceutically acceptable diluent or carrier, in combination with an effective amount of a 5-HT_{1B/1D} receptor agonist, or a pharmaceutically acceptable salt thereof, optionally together with a pharmaceutically acceptable diluent or carrier to a warm-blooded animal, such as man in need of such therapeutic treatment for use in the treatment of cancer.

The endothelin receptor antagonist, or a pharmaceutically acceptable salt thereof, will normally be administered to a warm-blooded animal at a unit dose of 1g or less daily but more than 2.5mg and this would be expected to provide a therapeutically-effective dose. However the daily dose will necessarily be varied depending upon the host treated, the particular route of administration, and the severity of the illness being treated. Accordingly the optimum dosage may be determined by the practitioner who is treating any particular patient. Particularly the endothelin antagonist could be administered to a warm-blooded animal, at a unit dose of less than 250 mg per day. In another aspect of the invention, the endothelin antagonist could be administered to a warm-blooded animal, at a unit dose of less than 130 mg per day. In a further aspect of the invention, the endothelin antagonist could be administered to a warm-blooded animal, at a unit dose of less than 50 mg per day.

The 5-HT_{1B/1D} receptor agonist, or pharmaceutically acceptable salt thereof, will normally be administered to a warm-blooded animal at a unit dose, for example, from about 0.5 mg to 15 mg (for example, 0.5 mg, 1.0 mg, 2.5 mg, 5.0 mg and 10 mg) of active ingredient. When the 5-HT_{1B/1D} receptor agonist is zolmitriptan, a conventional tablet formulation may be used for oral administration containing 2.5 mg or 5 mg of active ingredient. However the daily dose will necessarily be varied depending upon the host treated, the particular route of administration, and the severity of the illness being treated. Accordingly the optimum dosage may be determined by the practitioner who is treating any particular patient.

The dosage of each of the two drugs and their proportions have to be composed so that the best possible treatment effects, as defined by national and international guidelines (which are periodically reviewed and re-defined), will be met.

### Experimental

### Clinical study providing evidence of zolmitriptan being effective in the treatment of ZD4054 induced headache.

A study was undertaken to assess the safety and tolerability of multiple doses of ZD4054 in healthy young volunteers at dose levels of 100mg, 50mg and 25mg daily.

At the 100mg dose level 16 volunteers participated (12 active and 4 placebo). They received a 100mg ZD4054 or placebo single dose and one week later commenced daily dosing. Dosing was stopped within 3-5 days of continuous dosing in the majority of ZD4054 treated subjects due to headache. Of the 12 subjects receiving ZD4054, 10 were additionally given zolmitriptan (2.5 mg tablets) at some stage (in addition to paracetamol and in some cases ibuprofen).

Note: all subjects received paracetamol (1g every 4 hours for the first 16 hours post the first dose of ZD4054) on the first day of multiple dosing. Analgesics were given as required.

### Results

Seven patients noted at least at one stage during treatment that zolmitriptan alleviated their headache. Of these seven patients the following observations are noteworthy:
**Subject 7:** CTC grade 2 headache occurred on the second day of multiple dosing with little improvement with paracetamol. Zolmitriptan was then given (2.5mg) with a further 2.5mg 50 mins later and the headache lessened to CTC grade 1, 1-1.5 hours later.
**Subject 10:** At the multiple dose stage, following the first dose, a CTC grade 1 headache occurred. On second day the headache increased to CTC grade 3 despite paracetamol. Following administration of zolmitriptan (2.5mg) the headache reduced to CTC grade 2. On day 3 headache again increased to CTC grade 3. Zolmitriptan 2.5mg was given and a further 2.5mg 50 mins later. The headache reduced to CTC grade 1, 2.5h later.

An explanation of CTC grades which stands for Common Toxicity Criteria, can be found in in a document from the National Institute of Health, Version 2.0, DCTD, NCI, NIH, DHHS March 1998.

### Clinical study providing evidence of intranasal zolmitriptan being effective in the treatment of ZD4054 induced headache

The following double blind, placebo-controlled crossover study can be undertaken to assess the effectiveness of intranasal zolmitriptan compared to placebo in relieving headache induced by the acute administration of ZD4054 in healthy volunteers.

This study can also assess the effect of zolmitriptan on the incidence of nausea and/or vomiting following ZD4054 compared to placebo.

Twelve healthy male subjects will complete the study. The subjects will participate in two treatment periods with dosing separated by a minimum of 13 days. On each treatment period subjects will receive a single oral dose of 50mg ZD4054. Approximately 1 hour after the onset of headache the subjects will receive either 5mg intranasal zolmitriptan or matching placebo. The headache intensity and relief will be assessed using Visual Analogue Scales and Descriptive rating scales (Onset of Action of ibuprofen in the Treatment of Muscle-Contraction Headache; B Schachtel et al ; Headache 28:471-474, 1988; and Headache pain model for assessing and comparing the efficacy of over-the-counter analgesic agents: B Schachtel et al ; Clin Pharmacol Ther:50:322-329,1991). Additional relief medication (paracetamol) will be allowed 2 hours post the zolmitriptan/placebo treatment if needed. Subjects will receive the alternate treatment on their second treatment period.

### Assessments of effectiveness

Headache intensity during the study will be rated by volunteers on a 100mm visual analogue Pain Intensity Scale. Relief is defined as reduction to mild or no headache at two hours post administration of zolmitriptan.

The skilled person will understand that either of the two above studies could also be undertaken with another endothelin antagonist and / or 5-HT_{1B/1D} receptor agonist.

## Claims

1. The use of a 5-HT_{1B/1D} receptor agonist, or a pharmaceutically acceptable salt thereof, in the manufacture of a medicament for the treatment or prevention of headache that results from administering an endothelin receptor antagonist, or a pharmaceutically acceptable salt thereof, in a warm-blooded animal, such as man.

2. The use according to claim 1 wherein the 5-HT_{1B/1D} receptor agonist is selected from zolmitriptan, sumatriptan, eletriptan, frovatriptan, naratriptan, rizatriptan and almotriptan or a pharmaceutically acceptable salt thereof.

3. The use according to claims 1-2 wherein the 5-HT_{1B/1D} receptor agonist is zolmitriptan or a pharmaceutically acceptable salt thereof.

4. The use according to claims 1-3 wherein the endothelin receptor antagonist is selected from A-127722, atrasentan (ABT-627), BQ-123, BQ-788, BMS 182874, feloprentan, BSF 420627, FR139317, IPI-950, L-749,329, L-754,142, LU 110896, LU 110897, PD 156707, PD 155080, Ro 46-2005, bosentan (Ro 47-0203), SB 217242, SB 209670, TAK-044, YM598, sitaxsentan (TBC11251), ZD1611, ambrisentan, tezosentan, darusentan, N-[[2'-[[(4,5-dimethyl-3-isoxazolyl)amino] sulphonyl]-4-(2-oxazolyl)[1,1'-biphenyl]-2-yl]methyl]-*N*,3,3-trimethylbutanamide and *N*-(3-methoxy-5-methylpyrazin-2-yl)-2-(4-[1,3,4-oxadiazol-2-yl]phenyl)pyridine-3-sulphonamide (ZD4054) or a pharmaceutically acceptable salt thereof.

5. The use according to claims 1-4 wherein the endothelin receptor antagonist is selected from ZD4054.

6. A combination, comprising an endothelin receptor antagonist, or a pharmaceutically acceptable salt thereof, and a 5-HT_{1B/1D} receptor agonist, or a pharmaceutically acceptable salt thereof.

7. The combination according to claim 6 wherein the endothelin receptor antagonist is selected from A-127722, atrasentan (ABT-627), BQ-123, BQ-788, BMS 182874, feloprentan, BSF 420627, FR139317, IPI-950, L-749,329, L-754,142, LU 110896, LU 110897, PD 156707, PD 155080, Ro 46-2005, bosentan (Ro 47-0203), SB 217242, SB 209670, TAK-044, YM598, sitaxsentan (TBC11251), ZD1611, ambrisentan, tezosentan, darusentan, *N*-[[2'-[[(4,5-dimethyl-3-isoxazolyl)aminol sulphonyl]-4-(2-oxazolyl)[1,1'-biphenyl]-2-yl]methyl]-N,3,3-trimethylbutanamide and *N*-(3-methoxy-5-methylpyrazin-2-yl)-2-(4-[1,3,4-oxadiazol-2-yl]phenyl)pyridine-3-sulphonamide (ZD4054) or a pharmaceutically acceptable salt thereof.

8. The combination according to claims 6 or 7 wherein the 5-HT_{1B/1D} receptor agonist is selected from zolmitriptan, sumatriptan, eletriptan, frovatriptan, naratriptan, rizatriptan and almotriptan or a pharmaceutically acceptable salt thereof.

9. The combination according to any one of claims 6-8 wherein the endothelin receptor antagonist is ZD4054, or a pharmaceutically acceptable salt thereof, and the 5-HT_{1B/1D} receptor agonist is zolmitriptan, or a pharmaceutically acceptable salt thereof.

10. The combination according to any one of claims 6-9 for use as a medicament.

11. A pharmaceutical composition comprising the combination according to any one of claims 6-9, in association with a pharmaceutically acceptable diluent or carrier.

12. The use of the combination according to claims 6-9, in the manufacture of a medicament for use in the treatment of cancer, in a warm-blooded animal, such as man.

13. The use of the combination according to claims 6-9, in the manufacture of a medicament for use in the production of an anti-angiogenic effect, in a warm-blooded animal, such as man.

14. The use according to claim 12 wherein the cancer is oesophageal cancer, myeloma, hepatocellular, pancreatic, cervical cancer, ewings tumour, neuroblastoma, Kaposis sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, renal cancer lymphoma and leukaemia.

15. The use according to claim 12 wherein the cancer is prostate cancer.

16. The use according to any one of claims 12-15 wherein the cancer is in a metastatic state.

17. The use according to any one of claims 12-15 wherein the cancer is in a non-metastatic state.

18. The use according to any one of claims 12 - 15 wherein the cancer is renal, thyroid, lung, breast or prostate cancer that is producing bone metastases.

## Patentansprüche

1. Verwendung eines 5HT_{1B/1D}-Rezeptoragonisten oder eines pharmazeutisch annehmbaren Salzes davon bei der Herstellung eines Arzneimittels zur Behandlung oder Prävention von Kopfschmerzen, die von der Verabreichung eines Endothelinrezeptorantagonisten oder eines pharmazeutisch annehmbaren Salzes davon herrühren, bei einem warmblüter wie dem Menschen.

2. Verwendung nach Anspruch 1, bei der der 5HT_{1B/1D}-Rezeptoragonist unter Zolmitriptan, Sumatriptan, Eletriptan, Frovatriptan, Naratriptan, Rizatriptan und Almotriptan oder einem pharmazeutisch annehmbaren Salz davon ausgewählt ist.

3. Verwendung nach den Ansprüchen 1-2, bei der es sich bei dem 5HT_{1B/1D}-Rezeptoragonisten um Zolmitriptan oder ein pharmazeutisch annehmbares Salz davon handelt.

4. Verwendung nach den Ansprüchen 1-3, bei der der Endothelinrezeptorantagonist unter A-127722, Atrasentan (ABT-627), BQ-123, BQ-788, BMS 182874, Feloprentan, BSF 420627, FR139317, IPI-950, L-749,329, L-754,142, LU 110896, LU 110897, PD 156707, PD 155080, Ro 46-2005, Bosentan (Ro 47-0203), SB 217242, SB 209670, TAK-044, YM598, Sitaxsentan (TBC11251), ZD1611, Ambrisentan, Tezosentan, Darusentan, N-[[2'-[[(4,5-Dimethyl-3-isoxazolyl)amino]sulfonyl]-4-(2-oxazolyl)[1,1'-biphenyl]-2-yl]methyl]-N,3,3-trimethylbutanamid und N-(3-Methoxy-5-methylpyrazin-2-yl)-2-(4-[1,3,4-oxadiazol-2-yl]phenyl)pyridin-3-sulfonamid (ZD4054) oder einem pharmazeutisch annehmbaren Salz davon ausgewählt ist.

5. Verwendung nach den Ansprüchen 1-4, bei der der Endothelinrezeptorantagonist unter ZD4054 ausgewählt ist.

6. Kombination, enthaltend einen Endothelinrezeptorantagonisten oder ein pharmazeutisch annehmbares Salz davon und einen 5HT_{1B/1D}-Rezeptoragonisten oder ein pharmazeutisch annehmbares Salz davon.

7. Kombination nach Anspruch 6, in der der Endothelinrezeptorantagonist unter A-127722, Atrasentan (ABT-627), BQ-123, BQ-788, BMS 182874, Feloprentan, BSF 420627, FR139317, IPI-950, L-749,329, L-754,142, LU 110896, LU 110897, PD 156707, PD 155080, Ro 46-2005, Bosentan (Ro 47-0203), SB 217242, SB 209670, TAK-044, YM598, Sitaxsentan (TBC11251), ZD1611, Ambrisentan, Tezosentan, Darusentan, N-[[2'-[[(4,5-Dimethyl-3-isoxazolyl)amino]sulfonyl]-4-(2-oxazolyl)[1,1'-biphenyl]-2-yl]methyl]-N,3,3-trimethylbutanamid und N-(3-Methoxy-5-methylpyxazin-2-yl)-2-(4-[1,3,4-oxadiazol-2-yl]phenyl) pyridin-3-sulfonamid (ZD4054) oder einem pharmazeutisch annehmbaren Salz davon ausgewählt ist.

8. Kombination nach Anspruch 6 oder 7, in der der 5HT_{1B/1D}-Rezeptoragonist unter Zolmitriptan, Sumatriptan, Eletriptan, Frovatriptan, Naratriptan, Rizatriptan und Almotriptan oder einem pharmazeutisch annehmbaren Salz davon ausgewählt ist.

9. Kombination nach einem der Ansprüche 6-8, in der es sich bei dem Endothelinrezeptorantagonisten um ZD4054 oder ein pharmazeutisch annehmbares Salz davon und bei dem 5HT_{1B/1D}-Rezeptoragonisten um Zolmitriptan oder ein pharmazeutisch annehmbares Salz davon handelt.

10. Kombination nach einem der Ansprüche 6-9 zur Verwendung als Arzneimittel.

11. Pharmazeutische Zusammensetzung, enthaltend die Kombination nach einem der Ansprüche 6-9 zusammen mit einem pharmazeutisch annehmbaren Verdünnungsmittel oder Träger.

12. Verwendung der Kombination nach einem der Ansprüche 6-9 bei der Herstellung eines Arzneimittels zur Verwendung bei der Behandlung von Krebs bei einem warmblüter wie dem Menschen.

13. Verwendung der Kombination nach einem der Ansprüche 6-9 bei der Herstellung eines Arzneimittels zur Verwendung bei der Erzielung einer antiangiogenen Wirkung bei einem Warmblüter wie dem Menschen.

14. Verwendung nach Anspruch 12, bei der es sich bei dem Krebs um Speiseröhrenkrebs, Myelom, Leberzellkrebs, Bauchspeicheldrüsenkrebs, Zervixkrebs, Ewing-Sarkom, Neuroblastom, Kaposi-Sarkom, Eierstockkrebs, Brustkrebs, Kolorektalkrebs, Prostatakrebs, Blasenkrebs, Melanom, Lungenkrebs - nicht-kleinzelligen Lungenkrebs (NSCLC) und kleinzelligen Lungenkrebs (SCLC), Magenkrebs, Kopf- und Halskrebs, Nierenkrebs, Lamphom und Leukämie handelt.

15. Verwendung nach Anspruch 12, bei der es sich bei dem Krebs um Prostatakrebs handelt.

16. Verwendung nach einem der Ansprüche 12-15, bei der der Krebs sich in einem metastasierenden Stadium befindet.

17. Verwendung nach einem der Ansprüche 12-15, bei der der Krebs sich in einem nicht metastasierenden Stadium befindet.

18. Verwendung nach einem der Ansprüche 12-15, bei der es sich bei dem Krebs um Knochenmetasatasen produzierenden Nieren-, Schilddrüsen-, Lungen-, Brust- oder Prostatakrebs handelt.

## Revendications

1. Emploi d'un agoniste du récepteur 5-HT_{1B/1D}, ou d'un sel de qualité pharmaceutique dudit agoniste, dans la fabrication d'un médicament pour le traitement prophylactique ou thérapeutique des maux de tête résultant de l'administration d'un antagoniste du récepteur de l'endothéline, ou d'un sel de qualité pharmaceutique dudit antagoniste, chez un animal à sang chaud tel que l'homme.

2. Emploi selon la revendication 1 où l'agoniste du récepteur 5-HT_{1B/1D} est sélectionné parmi le zolmitriptane, le sumatriptane, l'eletriptane, le frovatriptane, le naratriptane, le rizatriptane et l'almotriptane, ou un sel de qualité pharmaceutique de l'un de ces composés.

3. Emploi selon les revendications 1-2 où l'agoniste du récepteur 5-HT_{1B/1D} est le zolmitriptane ou un sel de qualité pharmaceutique de ce composé.

4. Emploi selon les revendications 1-3 où l'antagoniste du récepteur de l'endothéline est sélectionné parmi A-127722, l'atrasentane (ABT-627), BQ-123, BQ-788, BMS 182874, le feloprentane, BSF420627, FR139317, IPI-950, L-749,329, L-754,142, LU 110896, LU 110897, PD 156707, PD 155080, Ro 46-2005, le bosentane (Ro 47-0203), SB 217242, SB 209670, TAK-044, YM598, le sitaxsentane (TBC11251), ZD 1611, l'ambrisentane, le tezosentane, le darusentane, le *N*-[[2'-[[(4,5-diméthy-3-isoxazolyl)amino]sulfonyl]-4-(2-oxazolyl)[1,1'-biphényl]-2-yl]méthyl]-N,3,3-triméthylbutanamide et le *N*-(3-méthoxy-5-méthylpyrazin-2-yl)-2-(4-[1,3,4-oxadiazol-2-yl]phényl)pyridine-3-sulfonamide (ZD4054) ou un sel de qualité pharmaceutique de l'un de ces composés.

5. Emploi selon la revendication 1-4 où l'antagoniste de récepteur de l'endothéline est sélectionné parmi ZD4054.

6. Combinaison, comprenant un antagoniste de récepteur de l'endothéline, ou un sel de qualité pharmaceutique dudit antagoniste, et un agoniste du récepteur 5-HT_{1B/1D}, ou un sel de qualité pharmaceutique dudit agoniste.

7. Combinaison selon la revendication 6 où l'antagoniste de récepteur de l'endothéline est sélectionné parmi A-127722, l'atrasentane (ABT-627), BQ-123, BQ-788, BMS 182874, le feloprentane, BSF 420627, FR139317, IPI-950, L-749,329, L-754,142, LU 110896, LU 110897, PD 156707, PD 155080, Ro 46-2005, le bosentane (Ro 47-0203), SB 217242, SB 209670, TAK-044, YM598, le sitaxsentane (TBC 11251), ZD 1611, l'ambrisentane, le tezosentane, le darusentane, le N-[[2'-[[(4,5-diméthy-3-isoxazolyl)amino]sulfonyl]-4-(2-oxazolyl)[1,1'-biphényl]-2-yl]méthyl]-*N*,3,3-triméthylbutanamide et le *N*-(3-méthoxy-5-méthylpyrazin-2-yl]-2-(4-[1,3,4-oxadiazol-2-yl]phényl)pyridine-3-sulfonamide (ZD4054) ou un sel de qualité pharmaceutique de l'un de ces composés.

8. Combinaison selon les revendications 6 ou 7 où l'agoniste du récepteur 5-HT_{1B/1D} est sélectionné parmi le zolmitriptane, le sumatriptane, l'eletriptane, le frovatriptane, le naratriptane, le rizatriptane et l'almotriptane, ou un sel de qualité pharmaceutique de l'un de ces composés.

9. Combinaison selon l'une quelconque des revendications 6 à 8 où l'antagoniste du récepteur de l'endothéline est ZD4054 ou un sel de qualité pharmaceutique dudit composé, et où l'agoniste du récepteur 5-HT_{1B/1D} est le zolmitriptane ou un sel de qualité pharmaceutique dudit composé.

10. Combinaison selon l'une quelconque des revendications 6 à 9 pouvant être employée en tant que médicament.

11. Composition pharmaceutique comprenant la combinaison selon l'une quelconque des revendications 6 à 9 associée à un diluant ou un vecteur de qualité pharmaceutique.

12. Emploi d'une combinaison selon les revendications 6 à 9 dans la fabrication d'un médicament pouvant être employé dans le traitement d'un cancer, chez un animal à sang chaud tel que l'homme.

13. Emploi de la combinaison selon les revendications 6 à 9 dans la fabrication d'un médicament destiné à produire un effet anti-angiogénique chez un animal à sang chaud tel que l'homme.

14. Emploi selon la revendication 12 où le cancer est un cancer de l'oesophage, un myélome, un cancer cervical, hépatocellulaire ou pancréatique, une tumeur d'Ewing, un neuroblastome, un sarcome de Kaposis, un cancer ovarien, un cancer du sein, un cancer colorectal, un cancer de la prostate, un cancer de la vessie, un mélanome, un cancer du poumon - cancer du poumon non à petites cellules (NSCLC) et cancer du poumon à petites cellules (SCLC) - un cancer gastrique, un cancer de la tête et du cou, un cancer rénal, un lymphome ou une leucémie.

15. Emploi selon la revendication 12 où le cancer est un cancer de la prostate.

16. Emploi selon l'une quelconque des revendications 12 à 15 où le cancer est dans un état métastatique.

17. Emploi selon l'une quelconque des revendications 12 à 15 où le cancer est dans un état non métastatique.

18. Emploi selon l'une quelconque des revendications 12 à 15 où le cancer et un cancer rénal, de la thyroïde, du poumon, du sein ou de la prostate qui produit des métastases osseuses.
